# EUROPEAN PATENT APPLICATION

(11) **EP 2 762 076 A1**
(43) Date of publication of application: **06.08.2014**
(21) Application number: 12835690.4
(22) Date of filing: 31.07.2012
(51) Int. Cl.: A61B 5/1459, A61B 5/00, A61B 5/07, G06Q 50/22

(54) **ANALYTE MONITORING SYSTEM**

(30) Priority: 27.09.2011 JP 2011210453
(71) Applicant: Terumo Kabushiki Kaisha, Tokyo 151-0072 (JP)
(72) Inventor: TORAI Yu, Ashigarakami-gun Kanagawa 259-0151 (JP); NOMURA Takafumi, Ashigarakami-gun Kanagawa 259-0151 (JP)
(74) Representative: Casalonga
(86) International application number: PCT/JP2012/069450
(87) International publication number: WO 2013/046911

(57) **Abstract**

A blood sugar monitoring system (10) as an analyte monitoring system is provided with a sensor (12) which detects a plurality of blood sugar levels in blood on the basis of a sampling interval, a meal data setting unit (48) which sets information based on meal contents, and a sampling interval setting unit (50) which sets the sampling interval of the sensor (12) using the information based on meal contents set by the meal data setting unit (48). By predicting a fluctuation curve of the blood sugar level after a meal by information based on meal contents to thereby change the sampling interval, a fluctuation state of the blood sugar level can be efficiently monitored.

## Description

### Technical Field

The present invention relates to an analyte monitoring system which detects an analyte on the basis of a sampling interval.

### Background Art

Diabetes leads to abnormal sugar, protein, fat metabolism or the like. In particular, accumulation and stagnation of blood glucose (blood sugar: analyte) continues. Therefore, a diabetic (hereinbelow, also referred to as a user) is required to continuously perform blood sugar management (blood sugar control). In recent years, in order to perform more advanced blood sugar management, there has been developed a continuous blood sugar monitoring system (analyte monitoring system) called CGM (Continuous Glucose Monitoring) which measures blood sugar levels at a predetermined sampling interval (refer to JP 2003-502090 W).

The blood sugar monitoring system disclosed in JP 2003-502090 W is provided with a sensor which measures a blood sugar level, a monitoring transmitter which transmits the detected measurement result, and a monitoring device (characteristic monitoring device) which receives the measurement result and displays the blood sugar level. The monitoring device is configured to obtain measurement results at a predetermined time interval by receiving signals from the monitoring transmitter at a preset sampling interval.

### Summary of Invention

Blood sugar level starts increasing from a mealtime. The fluctuation rate of the blood sugar level becomes large at a predetermined time before and after a peak value of the highest blood sugar level, and forms a fluctuation curve dropping with a relatively small fluctuation rate after a certain time has passed from the peak value. In blood sugar management, it is important to recognize a fluctuation state when the blood sugar level largely fluctuates after a meal to help treatment (guidance of meal contents and eating style, for example).

However, it is known that, since the ratio and the speed for various nutrients (carbohydrates, proteins, and fats, for example) taken through a meal to be convert to blood sugar are different from each other, a fluctuation state of the blood sugar level shows different fluctuation curves depending on meal contents. For example, in comparison between when taking a meal mainly containing carbohydrates and when taking a meal mainly containing fats, the blood sugar level increases faster and the fluctuation rate (fluctuation curve) thereof is larger when taking a meal mainly containing carbohydrates.

Therefore, in monitoring of blood sugar levels, it is desired to efficiently detect blood sugar levels according to the fluctuation curves of the blood sugar levels. However, in a conventional monitoring device (refer to JP 2003-502090 W), for example), since the blood sugar level is sampled at a fixed time interval, it is necessary to sufficiently make the sampling interval short in order to measure the blood sugar level in detail. In this case, even in a stable period during which the fluctuation rate of the blood sugar level is small (a period after a certain time has passed from a mealtime), a large number of pieces of blood sugar level data are detected. As a result, inconvenience such as an increase in the number of pieces of data handled by a system and an increase in the power consumption along with the detection occurs. On the other hand, when making the blood sugar level sampling interval long, disadvantageously, fluctuation of the blood sugar level may not be sufficiently grasped.

The present invention has been made in view of the above circumstances, and an object thereof is to provide an analyte monitoring system that can efficiently perform monitoring of the blood sugar level by making the sampling interval short in a period during which the blood sugar level (concentration of an analyte) largely fluctuates after a meal and making the sampling interval long in a period during which fluctuation of the blood sugar level is small, thereby performing excellent blood sugar management.

To achieve the aforesaid object, the present invention provides an analyte monitoring system that is provided with: a detection means which is embedded inside the body of a user and detects the concentration of an analyte on the basis of a sampling interval; a meal data setting means which sets information based on meal contents; and a sampling interval setting means which sets the sampling interval of the detection means using the information based on meal contents set by the meal data setting means, wherein the analyte monitoring system continuously measures an analyte by the detection means.

With the above configuration, since the sampling interval setting means sets the sampling interval of the detection means using information based on meal contents, it is possible to increase the number of times of blood sugar level (concentration of an analyte) sampling by making the sampling interval short in a period during which the analyte (blood sugar, for example) largely fluctuates after a meal and reduce the number of times of blood sugar level sampling by making the sampling interval long in a period during which fluctuation of the blood sugar level is small. That is, by using information based on meal contents, it is possible to relatively easily calculate a time for nutrients taken through a meal to be converted to blood sugar. As a result, it is possible to predict a fluctuation curve of the blood sugar level to thereby change the sampling interval. Therefore, a fluctuation state of the blood sugar level can be efficiently monitored, and excellent blood sugar management can thereby be performed.

In this case, it is preferred that the analyte monitoring system be further provided with a mealtime setting means which sets mealtime information, wherein the sampling interval setting means change the sampling interval of the detection means on the basis of mealtime information set by the mealtime setting means.

By timely changing the sampling interval of the detection means on the basis of mealtime information in this manner, the blood sugar level which increases after a meal and has a large fluctuation rate around a predetermined time near its peak value can be detected at a short sampling interval. As a result, it is possible to improve the monitoring accuracy during a period that is important for blood sugar management.

Further, the information based on meal contents may be a carbohydrate value indicating the amount of carbohydrates in each of the meal contents.

By using a carbohydrate value which indicates the amount of carbohydrates in each of the meal contents as the information based on meal contents, it becomes possible to predict a time for carbohydrates taken through a meal to be converted to blood sugar. Therefore, by setting the blood sugar level sampling interval from a carbohydrate value, it is possible to intensively measure blood sugar levels in a period during which the fluctuation rate is large.

Further, the analyte monitoring system may be provided with a database storage means which stores the carbohydrate value for each of the meal contents as a database, and a display means which displays a meal content with which the carbohydrate value is associated.

When a meal content with which a carbohydrate value is associated is displayed by the display means, a user can easily uses the carbohydrate value based on the displayed meal content.

Further, in response to selection of a meal content displayed by the display means, the meal data setting means may set the carbohydrate value of the selected meal content.

Accordingly, a user can use a carbohydrate value only by selecting a meal content displayed by the display means.

Therefore, it is possible to omit an operation of converting meal contents to carbohydrate values by a user him/herself, and thereby more easily set the blood sugar level sampling interval.

The analyte monitoring system may be further provided with a sampling pattern storage means which is capable of storing a plurality of pieces of pattern data combining a plurality of kinds of sampling intervals, wherein the sampling interval setting means can calculate a total value of carbohydrate values in one meal, and select one of the plurality of pieces of pattern data on the basis of the total value.

By selecting one of the plurality of pieces of pattern data on the basis of the total value of carbohydrate values in this manner, it is possible to easily change the sampling interval in accordance with the pattern data based on the carbohydrate value.

Further, it is preferred that the analyte be a blood sugar level, and, in each of the plurality of pieces of pattern data, a period during which each of the plurality of kinds of sampling intervals is performed may be set depending on the vicinity of a peak value in which the blood sugar level becomes highest after a meal on the basis of the carbohydrate value.

Since a period during which each of the plurality of kinds of sampling intervals is performed is set depending on the vicinity of a peak value in which the blood sugar level is predicted to be highest after a meal on the basis of the carbohydrate value in this manner, it is possible to detect the blood sugar level by setting the shortest sampling interval in one piece of pattern data so as to match a predetermined time before and after its peak value in which the fluctuation rate of the blood sugar level is large. As a result, it is possible to further improve the accuracy of blood sugar level monitoring.

According to the present invention, it is possible to efficiently perform monitoring of the blood sugar level by making the sampling interval short in a period during which the blood sugar level largely fluctuates after a meal and making the sampling interval long in a period during which fluctuation of the blood sugar level is small, thereby performing excellent blood sugar management.

### Brief Description of Drawings

Fig. 1 is a graph illustrating mealtime and fluctuation of the blood sugar level in a living body according to the present invention.
Fig. 2 is a table illustrating an example of the correspondence relationship between meal contents and carbohydrate values.
Fig. 3 is an explanatory diagram schematically illustrating the entire configuration of a blood sugar monitoring system according to the present embodiment.
Fig. 4 is a system block diagram of the blood sugar monitoring system of Fig. 3.
Fig. 5 is a functional block diagram of a monitoring device of Fig. 4 which performs processing for setting a sampling interval.
Fig. 6 is an explanatory diagram schematically illustrating sampling interval pattern data.
Fig. 7 is a flowchart illustrating an example of an operation of measuring the blood sugar level by the blood sugar monitoring system according to the present embodiment.
Fig. 8 is a flowchart illustrating an operation of processing the sampling interval by the blood sugar monitoring system according to the present embodiment.
Fig. 9A is a graph illustrating an example of a fluctuation curve of the blood sugar level according to a concrete example; and Fig. 9B is a sample graph of blood sugar level data obtained from the fluctuation curve of Fig. 9A when changing the blood sugar level sampling interval using the blood sugar monitoring system.

### Description of Embodiments

Hereinbelow, an analyte monitoring system (blood sugar monitoring system) according to the present invention will be described in detail by giving a preferred embodiment with reference to the accompanying drawings.

First, as an assumption for explaining the blood sugar monitoring system according to the present embodiment, the relationship between meal contents and fluctuation of the blood sugar level and the relationship between meal contents and a carbohydrate value will be described in detail. In the present embodiment, the "clock time" is used to indicate a certain point in the day such as 12:00 and 12:30, the "amount of time" is used to indicate the length of time from a certain point to another point such as one hour and one and a half hours, and the "time" is used so as to include the clock time and the amount of time.

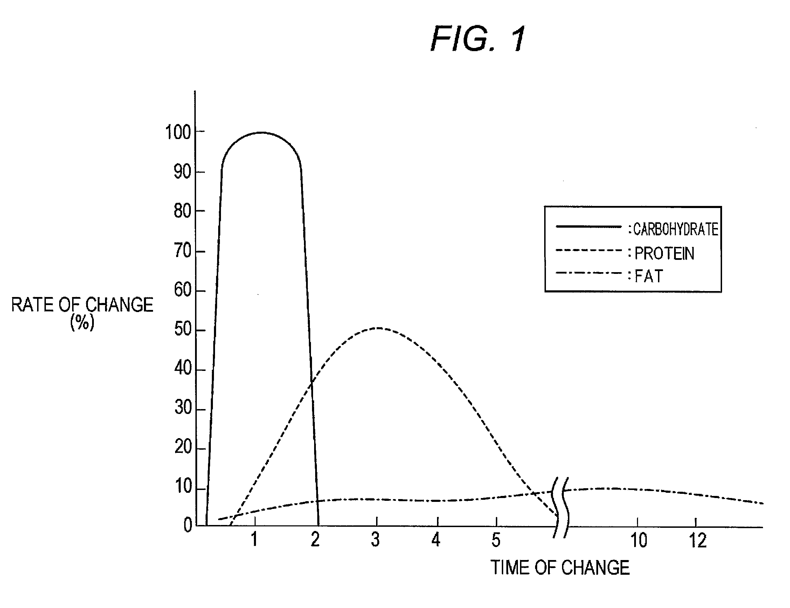

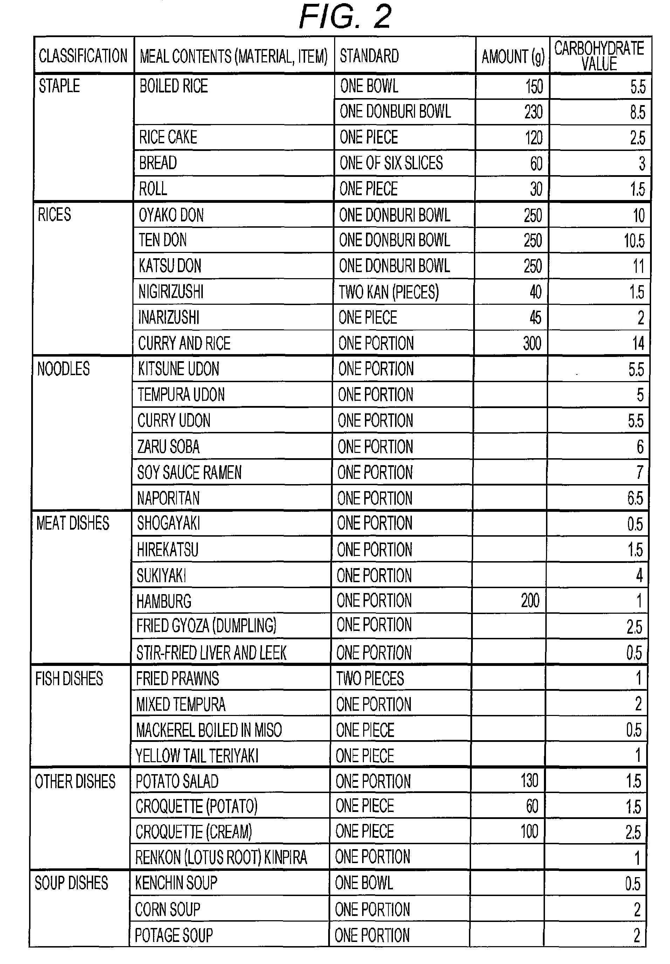

Fig. 1 is a graph illustrating mealtime and fluctuation of the blood sugar level in a living body according to the present invention. Fig. 2 is a table illustrating an example of the correspondence relationship between meal contents and carbohydrate values.

As illustrated in Fig. 1, it is known that, in a living body, the ratio and the time (namely, the speed) for nutrients taken through a meal to be converted to blood sugar (glucose) are different depending on meals. For example, the conversion rate of carbohydrates to glucose is approximately 100%, and carbohydrates can therefore be relatively easily digested. Therefore, the largest amount of carbohydrates is converted to glucose for approximately one hour after a meal. On the other hand, the conversion rate of proteins to glucose is approximately 50%, and proteins are converted to glucose gradually compared to carbohydrates. Therefore, the largest amount of proteins is converted to glucose for approximately three hours after a meal. Further, the conversion rate of fats to glucose is less than 10%, and fats are therefore take long time to be digested. Therefore, the peak of the amount of fats converted to glucose is at approximately 10 hours after a meal.

That is, the increasing speed of blood sugar level after a meal differs depending on the contents of the meal (nutrients in the meal). Therefore, it is difficult to set a time at which the blood sugar level increases to get its peak value after a meal. In conventional blood sugar management, a doctor often uniformly and experientially instructs to measure the blood sugar level at two hours after a meal.

In order to simply determine an increase in blood sugar level, a counting method using a carbohydrate value (carbohydrate counting) is conceivable. This carbohydrate counting is used when adjusting the dose of insulin which stabilizes blood sugar. The "carbohydrate value" is an index which indicates the amount of carbohydrates contained in meal contents. For example, 10 g of carbohydrates is defined as one carb, so that the carbohydrate value is calculated for each meal content.

For example, as in the table illustrated in Fig. 2, it is possible to calculate a carbohydrate value for each meal content in such a manner that the carbohydrate value of rice (150 g: one bowl) is 5.5, the carbohydrate value of bread (60 g: one of six slices) is 3.0, and the carbohydrate value of fried prawn (two pieces) is 1.0. When the carbohydrate value is high, the amount of carbohydrates taken in a meal is large, and the amount to be converted to blood sugar becomes large. In addition, it can be predicted that a time for the blood sugar level to reach to its peak value also becomes shorter.

The blood sugar monitoring system according to the present invention effectively utilizes the "carbohydrate value" which has been conventionally used only for adjusting the amount of insulin to thereby predict a fluctuation state of the blood sugar level (peak value and a fluctuation curve for a predetermined time around the peak value), thereby changing a blood sugar level sampling interval.

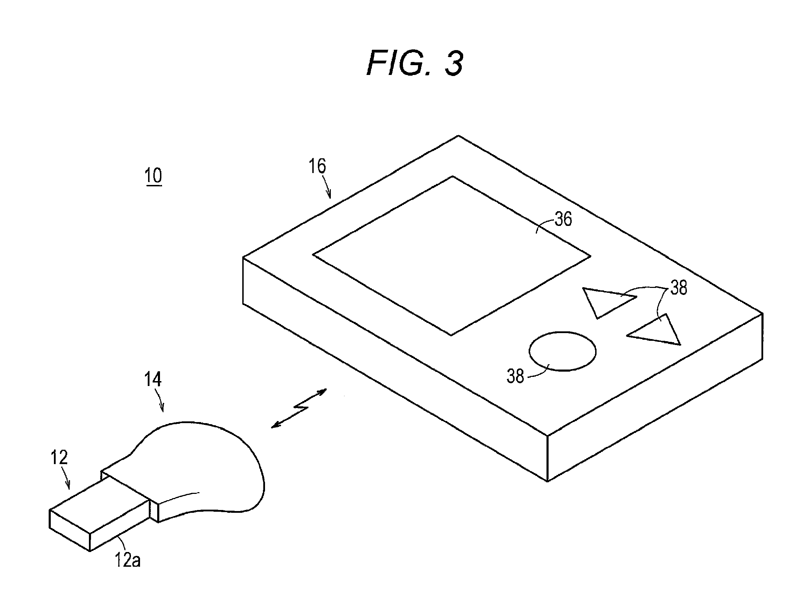

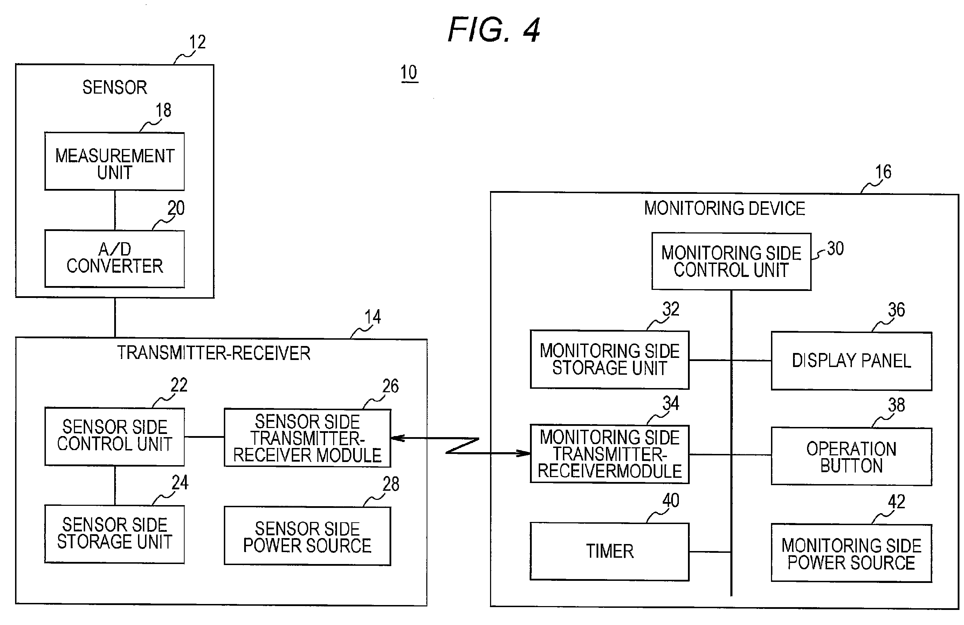

Fig. 3 is an explanatory diagram schematically illustrating the entire configuration of the blood sugar monitoring system 10 according to the present embodiment. Fig. 4 is a system block diagram of the blood sugar monitoring system 10 of Fig. 3.

As illustrated in Fig. 3, the blood sugar monitoring system 10 includes a sensor (detection means) 12 which measures a blood sugar level (concentration of an analyte), a transmitter-receiver 14 which wirelessly transmits a signal of the blood sugar level (measured value) measured by the sensor 12, and a monitoring device 16 which receives the signal transmitted by the transmitter-receiver 14 and stores or displays the measured value.

As the sensor 12, a sensing device that can optically measure blood glucose is applied. Specifically, the sensing device is a sensor (fluorescence sensor) for detection using a phenomenon in which the fluorescence intensity changes by causing the interaction between glucose and a labelled compound (fluorescent dye compound: for example, fluorescein labelled dextran or one having a fluorescence residue such as a phenyl boronic acid derivative).

A casing of the sensor 12 is formed into a generally rectangular shape using a resin material or the like which is capable of blocking outside light, and attached to a predetermined position (the arm or flank, for example) of a living body. A contact surface 12a which makes contact with a living body (skin) in the sensor 12 includes hydrogel, carbon black, and the like. Accordingly, the contact surface 12a allows glucose to pass therethrough, and, on the other hand, blocks outside light.

As illustrated in Fig. 4, a measurement unit 18 and an A/D converter 20 are provided inside the casing of the sensor 12. In the measurement unit 18, a base which is made of silicone or the like, a light receiving element (a photodiode element, for example), a protection film, a filter, a light emitting element (a light emitting diode element, for example), an indicator layer, and the like are laminated. Further, a puncture needle is provided on the side of the contact surface 12a which is continuous with the indicator layer (both not illustrated).

The measurement unit 18 guides blood to the indicator layer through the contact surface 12a when the puncture needle is stuck and indwelled (that is, embedded) in a living body. The indicator layer includes fluorescent dye as a labelled compound. Accordingly, glucose that has entered the indicator layer through the contact surface 12a and the labelled compound interact each other. The sensor 12 allows the light emitting element of the measurement unit 18 to emit light, and thereby allows measurement light to enter the indicator layer to obtain fluorescent light having an intensity corresponding to the concentration of glucose. The fluorescent light from the indicator layer passes through the filter and the like, then photoelectrically converted by the light receiving element, and then supplied to the A/D converter 20 as a signal of blood sugar level.

Although, as the sensor 12, it is preferred to apply an optical sensor (the fluorescence sensor as described above) that can easily change a blood sugar level sampling interval, the sensor 12 is not limited to the optical sensor. For example, a sensor that electrically measures blood sugar levels by an oxygen electrode method (electrochemical method) or the like using an enzyme such as glucose oxidase (GOD) may also be applied.

The A/D converter 20 converts a current value (analog signal) of the blood sugar level detected by the measurement unit 18 into a voltage value, and amplifies the voltage value so as to be converted to a digital signal. Accordingly, a digital signal of the blood sugar level is sent from the sensor 12 to the transmitter-receiver 14.

As illustrated in Fig. 3, the transmitter-receiver 14 of the blood sugar monitoring system 10 is directly connected to one end of the sensor 12, and has functions to control blood sugar level detection by the sensor 12 and transmit the blood sugar level detected by the sensor 12 to the monitoring device 16. The transmitter-receiver 14 is formed into a flat casing so as to be easily placed on the skin of a living body together with the sensor 12. As illustrated in Fig. 4, a sensor side control unit 22, a sensor side storage unit 24, a sensor side transmitter-receiver module 26, a sensor side power source 28, and the like are provided inside the casing of the transmitter-receiver 14.

A well-known microcomputer (microprocessor: MPU) or the like is applied as the sensor side control unit 22 of the transmitter-receiver 14. The sensor side control unit 22 operates according to a program (not illustrated) for measurement stored in the sensor side storage unit 24 to thereby control blood sugar level detection by the sensor 12. The sensor side control unit 22 has an internal timer (not illustrated) which utilizes a processing clock, and performs blood sugar level detection based on the sampling interval by counting by the timer.

The sensor side storage unit 24 includes a ROM and a RAM. A program for measuring blood sugar levels is previously stored in the ROM. In addition, blood sugar level data detected by the sensor 12 and sampling interval information (described below) are temporarily stored in the RAM.

The sensor side transmitter-receiver module 26 has a function to transmit/receive data to/from a monitoring side transmitter-receiver module 34 of the monitoring device 16. In particular, in the present embodiment, a module that achieves wireless data communication (an RF module which can output and input radio waves in a high frequency band, for example) is applied as the sensor side transmitter-receiver module 26. When performing blood sugar monitoring, it is predicted that an arrangement position of the monitoring device 16 is not largely separated from arrangement positions of the sensor 12 and the transmitter-receiver 14 (that is, the location of a living body). Therefore, a short-range communication standard (IEEE 802.15, for example) may be employed in the sensor side transmitter-receiver module 26 and the monitoring side transmitter-receiver module 34.

For example, a button type battery, a round dry battery, a square dry battery, a secondary battery, or an external power source can be applied as the sensor side power source 28. The sensor side power source 28 supplies necessary electric power to the sensor 12 and internal mechanisms (the measurement unit 18, the A/D converter 20, the sensor side control unit 22, the sensor side storage unit 24, the sensor side transmitter-receiver module 26, and the like) of the transmitter-receiver 14, the internal mechanisms being driven by electric power.

On the other hand, as illustrated in Fig. 3, the monitoring device 16 of the blood sugar monitoring system 10 is configured as a portable terminal in which a display panel (display means) 36 and an operation button 38 are provided. As illustrated in Fig. 4, a monitoring side control unit 30, a monitoring side storage unit 32, the monitoring side transmitter-receiver module 34, a timer 40, a monitoring side power source 42, and the like are arranged inside a casing of the monitoring device 16.

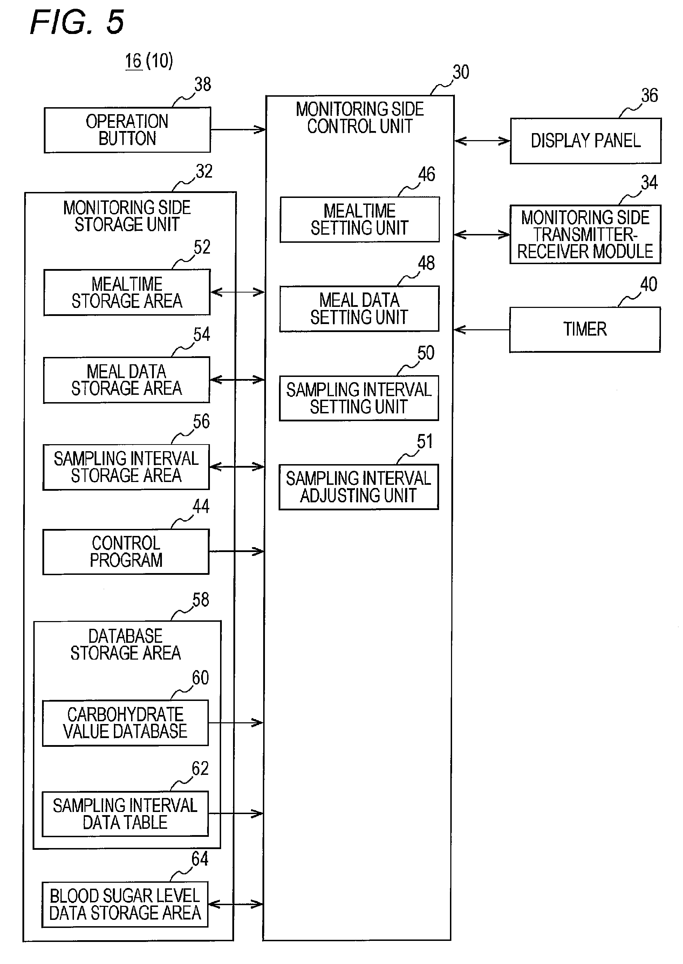

In the same manner as in the sensor side control unit 22, a well-known microcomputer or the like is applied as the monitoring side control unit 30. The monitoring side control unit 30 performs predetermined processing on the basis of a program for control (hereinbelow, referred to as a control program 44: refer to Fig. 5) which is previously stored in the monitoring side storage unit 32.

The control program 44 controls the entire blood sugar monitoring system 10 including the sensor 12, the transmitter-receiver 14, and the monitoring device 16. More specifically, the monitoring device 16 can perform, on the basis of the control program 44, setting of an interval of blood sugar level sampling performed by the sensor 12, storage and display of data of a plurality of blood sugar levels detected by the sensor 12, transmittance of blood sugar level data to an external device (a computer, for example) and the like.

In the same manner as in the sensor side storage unit 24, the monitoring side storage unit 32 includes a ROM and a RAM. In the monitoring side storage unit 32, the control program 44 is previously stored in the ROM, and a plurality of data areas for storing various pieces of data measured by the sensor 12 are allocated in an address space on the RAM.

Further, the monitoring side transmitter-receiver module 34 has a function to transmit and receive predetermined data on the basis of an operation of the monitoring side control unit 30 (or the sensor side control unit 22) . The monitoring side transmitter-receiver module 34 performs wireless data communication with the sensor side transmitter-receiver module 26, and also performs wireless data transmission with the external device. It is needless to say that the transmission /reception of data between the transmitter-receiver 14 and the monitoring device 16 is not limited to wireless communication, and may also be wire communication.

The display panel 36 is arranged on the upper surface of the casing of the monitoring device 16 so as to have a relatively large display area (refer to Fig. 3). The display panel 36 includes, for example, a liquid crystal monitor, an organic electroluminescence (EL), and an inorganic EL. The display panel 36 is connected to internal mechanisms of the monitoring device 16, and displays information required for blood sugar level monitoring (a blood sugar level, date and time, a sampling interval, an operation procedure, and an error, for example) on the basis of control by the monitoring side control unit 30.

On the other hand, the operation button 38 includes a plurality of pressing-type buttons which are arranged on the upper surface of the casing together with the display panel 36. Accordingly, a user can press the operation button 38 while confirming information displayed on the display panel 36.

It is needless to say that the monitoring device 16 is not limited to the above configuration that is provided with the display panel 36 and the operation button 38. For example, display and operations required in blood sugar level monitoring may be integrally performed by employing a touch panel type display panel.

Further, the timer 40 is provided for managing clock time (date and time) inside the monitoring device 16. The timer 40 automatically measures clock time regardless of ON/OFF of the power source of the monitoring device 16.

In the same manner as in the sensor side power source 28, various power sources can be applied as the monitoring side power source 42. The monitoring side power source 42 supplies necessary electric power to internal mechanisms (the monitoring side control unit 30, the monitoring side storage unit 32, the monitoring side transmitter-receiver module 34, the display panel 36, the timer 40, and the like) of the monitoring device 16, the internal mechanisms being driven by electric power.

The blood sugar monitoring system 10 links the above configurations to each other to set a blood sugar level sampling interval, and discretely detects a plurality of blood sugar levels on the basis of the set sampling interval. In particular, in the setting of the sampling interval, a period during which the blood sugar level largely fluctuates after a meal is predicted using the above-described carbohydrate value, and the sampling interval in the predicted period is made short to increase the number of times of blood sugar level sampling, thereby accurately grasping blood sugar level fluctuation. The processing for setting a sampling interval is performed by the monitoring device 16 (the control program 44).

Fig. 5 is a functional block diagram of the monitoring device 16 of Fig. 4 which performs processing for setting the sampling interval.

As described above, the control program 44 is previously stored in the monitoring side storage unit 32 of the monitoring device 16. The blood sugar monitoring system 10 performs, for example, setting of mealtime and meal contents, setting of a blood sugar level sampling interval, and display and storage of blood sugar levels detected by the sensor 12 by the monitoring side control unit 30 operating according to the control program 44. The blood sugar monitoring system 10 is not limited to the above configuration, and may be configured as an integrated apparatus in which the sensor 12, the transmitter-receiver 14, and the monitoring device 16 are integrated. Further, the setting of a blood sugar level sampling interval or the like is not necessarily performed inside the monitoring device 16, and may be performed inside the sensor 12 or the transmitter-receiver 14, for example.

In this case, the display panel 36 of the monitoring device 16 can display information required for measurement of blood sugar levels by the blood sugar monitoring system 10 (mealtime, meal contents, a carbohydrate value, and a sampling interval, for example) on the basis of control by the monitoring side control unit 30 which operates according to the control program 44. Similarly, the operation button 38 also can perform various operations for setting information required for measurement of blood sugar levels.

In the monitoring device 16, on the basis of the control program 44, a mealtime setting unit 4 6 (mealtime setting means), a meal data setting unit 48 (meal data setting means), a sampling interval setting unit 50 (sampling interval setting means) and a sampling interval adjusting unit 51 are provided inside the monitoring side control unit 30. Corresponding to this, a mealtime storage area 52, a meal data storage area 54, and a sampling interval storage area 56 are formed in a data area of the monitoring side storage unit 32. Further, in the monitoring side storage unit 32, a carbohydrate value database 60 and a sampling interval data table 62 each of which is necessary information in the setting of the sampling interval are previously stored in a database storage area 58 (database storage means, sampling interval storage means).

The mealtime setting unit 46 stores (sets) mealtime information that has been input on the basis of an operating instruction by a user in the mealtime storage area 52. Here, the "mealtime information" is data regarding a time when a user takes a meal, and the user inputs the time to the monitoring device 16 as the mealtime information after (or before) taking the meal. When a user inputs mealtime information, the mealtime information may be input as a clock time such as 12:00 and 12: 30. On the other hand, the mealtime information may also be input as the amount of time such as one hour later and one and a half hours later with respect to a current clock time measured by the timer 40. However, the mealtime information is preferably input as information of clock time in the mealtime storage area 52 so as to easily have a correspondence relationship with a clock time measured by the timer 40.

The blood sugar monitoring system 10 according to the present embodiment predicts (sets) a fluctuation state of the blood sugar level on the basis of the time after a meal. Therefore, it is possible to improve the measurement accuracy when setting the mealtime information after a meal. The mealtime information may, of course, be set by predicting the finish time of a meal. Further, in a case where taking a meal does not take so long time, even if a clock time before and during a meal is set as the mealtime information, an error does not become large.

In the setting of mealtime information, since clock time is measured by the timer 40, a user operates the operation button 38 on the basis of the clock time of the timer 40 to thereby appropriately input mealtime information so as to be stored in the mealtime storage area 52. Further, the monitoring device 16 may be provided with a dedicated button for inputting clock time, and the dedicated button may be pressed once after finishing a meal to automatically set a clock time at which the dedicated button is pressed as the mealtime information. Further, when a clock time for taking a meal in each day is in substantially the same time zone, the mealtime may be previously registered (stored) in the mealtime storage area 52. In this case, a plurality of pieces of mealtime information such as breakfast, lunch, and dinner can also be stored in the mealtime storage area 52. When the mealtime has come (or approaches), the monitoring side control unit 30 may notify a user that the mealtime has come on the basis of a clock time measured by the timer 40 and the mealtime information previously stored to urge the user to input a carbohydrate value.

The mealtime information stored in the mealtime storage area 52 is read out when the monitoring side control unit 30 sets a blood sugar level sampling interval, and used as data for calculation.

On the other hand, the meal data setting unit 48 stores (sets) information based on meal contents input on the basis of an operating instruction by a user in the meal data storage area 54. Here, as the "information based on meal contents", a carbohydrate value which indicates the amount of carbohydrates in each of meal contents (described in the assumption of the present invention) is used.

More specifically, the meal data setting unit 48 stores a carbohydrate value as numerical data in the meal data storage area 54 when a user operates the operation button 38. In this case, carbohydrate values for respective meal contents (items) in one meal are added by a user, and the added value is stored in the meal data storage area 54 as a total value. Further, the monitoring device 16 may have a calculation function (not illustrated), and calculate a total value of carbohydrate values when a user inputs carbohydrate values for respective meal contents. Further, carbohydrate values for respective meal contents may be stored in association with mealtime information stored in the mealtime storage area 52. Also in this case, the monitoring side control unit 30 can predict a fluctuation state of the blood sugar level by collectively reading out and adding meal contents (carbohydrate values) taken in the same clock time (time zone).

As described above, the carbohydrate value database 60 is previously stored in the database storage area 58 of the monitoring side storage unit 32. The carbohydrate value database 60 is, for example, a plurality of data groups in which carbohydrate values are given for respective meal contents as described in Fig. 2. The monitoring side control unit 30 reads out the carbohydrate value database 60 on the basis of an operation by a user, and displays the read-out carbohydrate value database 60 on the display panel 36. Accordingly, a user can input a carbohydrate value to the meal data storage area 54 while confirming the carbohydrate value by the displayed carbohydrate value database 60.

As a method for displaying meal contents using the carbohydrate value database 60, for example, a tree form may be employed. In the tree form, a broad classification illustrated in Fig. 2 (staple, rices, noodles, and the like) is first selected, and a detailed classification (rice, rice cake, bread, and the like) is further selected.

The meal data setting unit 48 may allow a user to select a meal content (the carbohydrate value database 60) displayed on the display panel 36 without directly inputting a carbohydrate value by a user, and store a carbohydrate value of the selected meal content in the meal data storage area 54. In this case, when a touch panel is employed as the display panel 36, it is possible to allow a user to select a plurality of meal contents registered in the carbohydrate value database 60 while displaying the meal contents on the display panel 36. As a result, input of data can be made easier.

In addition to meal contents and carbohydrate values, picture data associated with the meal contents may be stored in the monitoring side storage unit 32 (the database storage area 58, for example). The picture data associated with the meal contents are displayed by the display panel 36, thereby making it possible to make input (selection) of a carbohydrate value for each meal content by a user further easier.

The sampling interval setting unit 50 has a function to read out the mealtime information set by the mealtime setting unit 46 from the mealtime storage area 52 and the carbohydrate value set by the meal data setting unit 48 from the meal data storage area 54 to set a blood sugar level sampling interval. The sampling interval information set by the sampling interval setting unit 50 is stored in the sampling interval storage area 56.

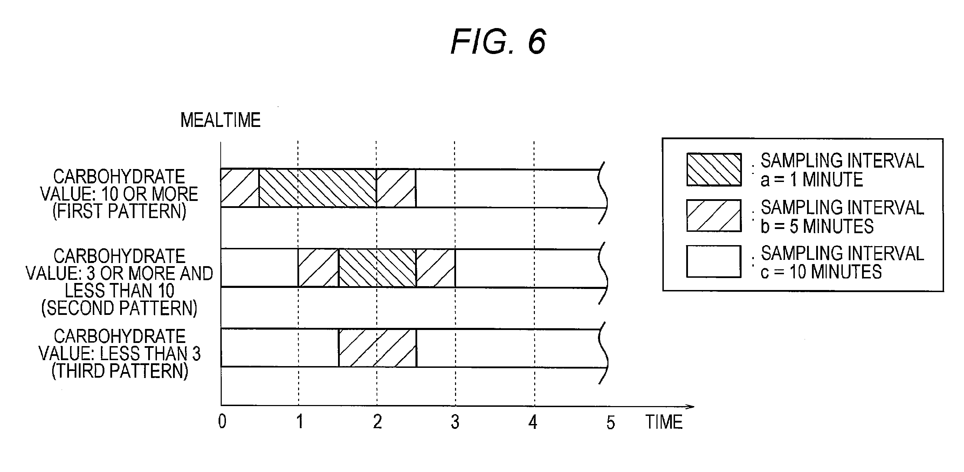

The blood sugar level sampling interval according to the present embodiment is set on the basis of the table (sampling interval data table 62) stored in the database storage area 58. The sampling interval data table 62 is prepared as a plurality of pieces of pattern data corresponding to carbohydrate values. Fig. 6 is an explanatory diagram schematically illustrating an example of the sampling interval pattern data. In Fig. 6, for the purpose of easy understanding of a sampling interval pattern, the pattern data is represented by a horizontal bar graph. However, the pattern data may, of course, be simply stored as numerical data in the database storage area 58.

As illustrated in Fig. 6, in the sampling interval data tale 62, a plurality of sampling intervals (three sampling intervals in Fig. 6) are set. Specifically, in a part indicated by hatching with a large number of oblique lines in Fig. 6, the sampling interval is set at one minute (hereinbelow, also referred to as a sampling interval a). In a part indicated by hatching with a small number of oblique lines in Fig. 6, the sampling interval is set at five minutes (hereinbelow, also referred to as a sampling interval b). In a part without hatching in Fig. 6, the sampling interval is set at ten minutes (hereinbelow, also referred to as a sampling interval c). Detection of blood sugar levels by the sensor 12 is performed on the basis of one piece of pattern data constructed by appropriately combining the three sampling intervals a to c.

In the sampling interval data table 62, the range of carbohydrate values (total value in one meal) is divided into three including: 10 or more, three or more and less than 10, and less than 3. Three pieces of pattern data are set so as to correspond to the respective three ranges (hereinbelow, also referred to as a first pattern, a second pattern, and a third pattern respectively corresponding to the three ranges of carbohydrate values (10 or more, 3 or more and less than 10, and less than 3)). The pattern data is set as the amount of time based on (zero base) mealtime (mealtime information). That is, the time scale in the horizontal axis in Fig. 6 represents one hour after a meal, two hours after a meal, and so on with mealtime as a base point.

In the first pattern in which the range of carbohydrate values is 10 or more, the sampling interval b of five minutes is started immediately after the mealtime (zero time). After 30 minutes, the sampling interval b is switched to the sampling interval a of one minute. Then, after performing the sampling interval a for 90 minutes, the sampling interval a is again switched to the sampling interval b, and the sampling interval b is performed for 30 minutes. Thereafter, the sampling interval c of 10 minutes is continuously performed. That is, when the carbohydrate value is 10 or more, it is assumed that the blood sugar level relatively rapidly increases after the meal, and reaches its peak value at approximately one hour after the meal. Therefore, in the first pattern, in order to accurately detect the fluctuation rate of the blood sugar level around one hour after a meal, a period during which the sampling interval a is performed is set so as to include one hour after the meal.

On the other hand, in the second pattern in which the range of carbohydrate values is 3 or more and less than 10, the sampling interval c of ten minutes is performed immediately after the mealtime (zero time). Then, the sampling interval c is switched to the sampling interval b of five minutes at one hour after the meal, and the sampling interval b is performed for 30 minutes. Then, the sampling interval b is switched to the sampling interval a of one minute, and the sampling interval a is performed for 60 minutes. Thereafter, the sampling interval a is again switched to the sampling interval b, and the sampling interval b is performed for 30 minutes. Finally, the sampling interval b returns to the sampling interval c, and the sampling interval c is continuously performed. That is, when the carbohydrate value is 3 or more and less than 10, it is assumed that the blood sugar level gradually increases after the meal, and reaches its peak value at approximately two hours after the meal. Therefore, in the second pattern, in order to accurately detect the fluctuation rate of the blood sugar level around two hours after a meal, a period during which the sampling interval a is performed is set so as to include two hours after the meal.

Further, in the third pattern in which the range of carbohydrate values is less than 3, the sampling interval c of ten minutes is performed immediately after the mealtime (zero time) in the same manner as in the second pattern. Then, the sampling interval c is switched to the sampling interval b of five minutes at one and a half hours after the meal, and the sampling interval b is performed for 60 minutes. Thereafter, the sampling interval b again returns to the sampling interval c, and the sampling interval c is continuously performed. That is, when the carbohydrate value is less than 3, it is assumed that a peak value of the blood sugar level does not become so large after the meal, and change of the blood sugar level forms a relatively low mountain shape. Therefore, in the third pattern, the sampling interval b is performed around two hours after the meal without performing the sampling interval a.

Referring back to Fig. 5, the sampling interval setting unit 50 of the monitoring device 16 selects one piece of pattern data from the pattern data (the first to third patterns) of the sampling interval data table 62 on the basis of a carbohydrate value. Then, the selected piece of pattern data is converted to a clock time on the basis of mealtime information (mealtime), and stored (set) as information of blood sugar level the sampling interval in the sampling interval storage area 56.

The sampling interval setting unit 50 may manually change pieces of pattern data on the basis of an operating instruction by a user, and store the changed piece of pattern data in the sampling interval storage area 56. For example, a carbohydrate value may be unsure in some meal contents, and the sampling interval (pattern data) may not therefore be set using a carbohydrate value. Therefore, by setting the sampling interval by an operating instruction by a user, it is possible to further improve versatility.

Further, the sampling interval adjusting unit 51 has a function to change the blood sugar level sampling intervals a to c stored in the sampling interval data table 62 on the basis of an operating instruction by a user. In other words, the blood sugar level sampling intervals a to c according to the present embodiment are not fixed parameters, but can be adjusted by an operation by a user.

For example, in the sampling intervals a to c illustrated in Fig. 6, adjustment of the interval of time for detecting blood sugar levels is conceivable. Specifically, the sampling interval a of one minute is adjusted in the range of approximately 1 to 2 minutes, the sampling interval b of five minutes is adjusted in the range of approximately 3 to 8 minutes, and the sampling interval c of ten minutes is adjusted in the range of approximately 8 to 15 minutes. By adjusting the blood sugar level sampling intervals by the sampling interval adjusting unit 51 in this manner, the number of times of sampling can be adjusted depending on the state of a patient (the condition of the disease, with or without exercise, and the like), for example. As a result, blood sugar level detection with high flexibility can be achieved.

The sampling interval adjusting unit 51 simultaneously rewrites the pattern data of the sampling interval data table 62 by storing (setting) the sampling intervals a to c adjusted by a user in the database storage area 58. Accordingly, when one piece of the pattern data (the first to third patterns) is selected by using a carbohydrate value, the adjusted sampling interval is used. The range of the adjustment of the sampling intervals is, of course, not limited to the above range.

The monitoring side transmitter-receiver module 34 of the monitoring device 16 timely and wirelessly transmits sampling interval information (data) stored in the sampling interval storage area 56 to the sensor side transmitter-receiver module 26 of the transmitter-receiver 14 (refer to Fig. 4) on the basis of control by the monitoring side control unit 30. In this case, the monitoring device 16 transmits the sampling interval information to the transmitter-receiver 14 at the timing when switching a currently-performed sampling interval to a different sampling interval (when switching the sampling interval a to the sampling interval b, for example) on the basis of measurement of clock time by the timer 40.

As illustrated in Fig. 4, when the transmitter-receiver 14 receives sampling interval information from the monitoring device 16, the transmitter-receiver 14 stores the sampling interval information in the sensor side storage unit 24. Then, the sensor side control unit 22 changes a blood sugar level sampling interval on the basis of the sampling interval information to control blood sugar level detection by the sensor 12.

Data of the blood sugar level detected on the basis of the sampling interval information is temporality stored in the sensor side storage unit 24 of the transmitter-receiver 14. Then, the sensor side control unit 22 transmits the data of the detected blood sugar level to the monitoring device 16 (the monitoring side transmitter-receiver module 34) through the sensor side transmitter-receiver module 26. In this case, every time when the sensor 12 detects a blood sugar level, data of the detected blood sugar level may be transmitted from the transmitter-receiver 14 to the monitoring device 6. Alternately, a plurality of pieces of blood sugar level data may be stored in the transmitter-receiver 14, and collectively transmitted to the monitoring device 16.

The monitoring device 16 stores blood sugar level data in the blood sugar level data storage area 64 of the monitoring side storage unit 32, and displays the stored blood sugar level on the display panel 36, for example, in response to an operation by a user. Examples of a method for displaying blood sugar levels by the display panel 36 includes various display methods such as continuously displaying blood sugar level data as numerical data, displaying blood sugar levels as a sample graph, and displaying a fluctuation curve of the blood sugar level by predicting continuous (analog) blood sugar levels from the sampling interval and blood sugar level data.

Next, setting of the sampling interval in the monitoring device 16 will be specifically described. The monitoring side control unit 30 operates each of the setting units 46 and 48 by performing processing according to the control program 44, and stores each piece of setting information input by a user in each of the areas 52 and 54 of the monitoring side storage unit 32. The sampling interval setting unit 50 (monitoring side control unit 30) can relatively easily calculate a time near a peak value of the blood sugar level on the basis of mealtime information stored in the monitoring side storage unit 32 and a carbohydrate value (information based on meal contents).

Specifically, the sampling interval setting unit 50 reads out a carbohydrate value corresponding to mealtime information from the meal data storage area 54. At this point, when carbohydrate values are separately stored for respective meal contents, all of these carbohydrate values are added to calculate a total value thereof. When carbohydrate values are stored as a total value in the meal data storage area 54, the stored values is simply read out.

Then, the sampling interval setting unit 50 reads out the sampling interval data table 62 previously stored in the database storage area 58. Then, the sampling interval setting unit 50 selects any one piece of pattern data corresponding to the carbohydrate value (total value) from data of the first to third patterns registered in the sampling interval data table 62. Then, the sampling interval setting unit 50 reads out mealtime information from the mealtime storage area 52, matches a zero base of the selected piece of pattern data to the set mealtime, and stores (sets) blood sugar level sampling interval information based on clock time in the sampling interval storage area 56.

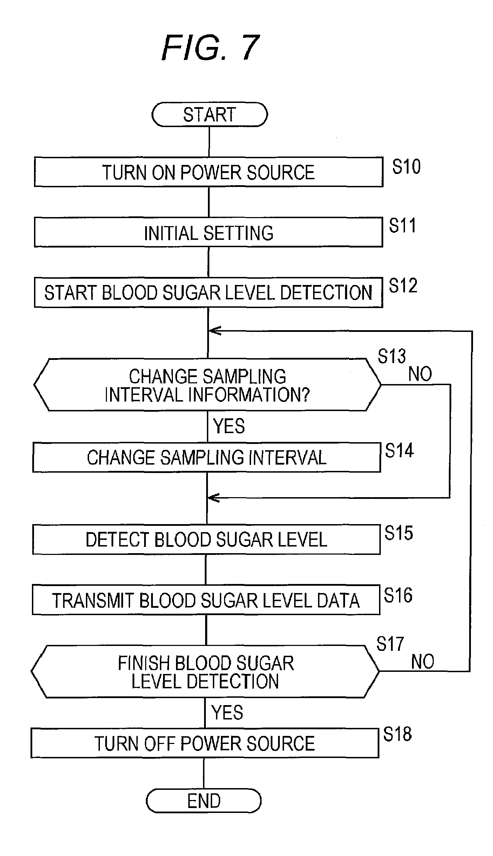

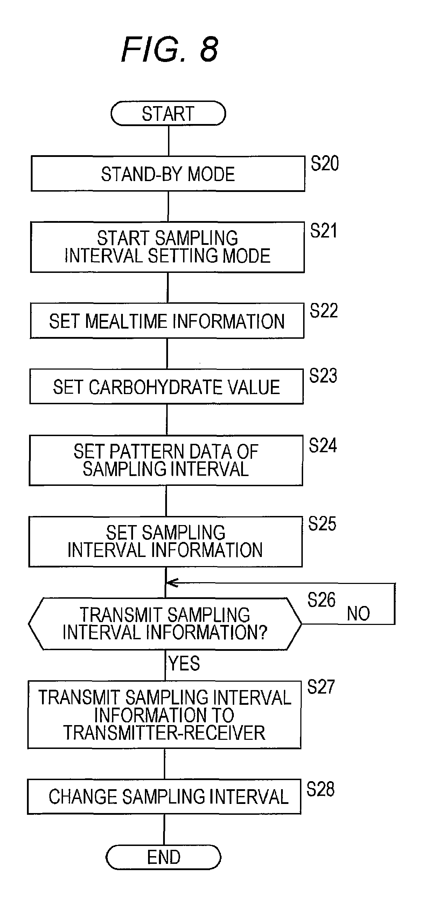

The blood sugar monitoring system 10 is basically configured as described above. Next, an operation and an effect of the blood sugar monitoring system 10 will be described. Fig. 7 is a flowchart illustrating an example of an operation of measuring the blood sugar level by the blood sugar monitoring system 10 according to the present embodiment. Fig. 8 is a flowchart illustrating an operation of processing the sampling interval by the blood sugar monitoring system 10 according to the present embodiment.

When measuring the blood sugar level using the blood sugar monitoring system 10 according to the present embodiment, a doctor or a nurse first attaches the sensor 12 and the transmitter-receiver 14 to a predetermined measurement position (the flank or upper arm, for example) of a patient (user). In this case, the puncture needle of the sensor 12 is stuck under the skin of the user to set the sensor 12 so as to direct blood of the user to the contact surface 12a of the sensor 12. Accordingly, in the blood sugar monitoring system 10, placement of the mechanical devices is completed, and measurement of a blood sugar level can be performed.

After placing the devices, a doctor or a nurse turns on the power sources (the sensor side power source 28 and the monitoring side power source 42) of the blood sugar monitoring system 10, and the sensor 12, the transmitter-receiver 14 and the monitoring device 16 are thereby actuated (step S10).

Then, initial setting of blood sugar level measurement by the blood sugar monitoring system 10 is performed through the monitoring device 16 (step S11). In the initial setting, setting of a blood sugar level sampling interval in a normal state without mealtime is performed in the monitoring device 16, and the sampling interval information in the initial setting is transmitted to the transmitter-receiver 14. In the present embodiment, the sampling interval in the normal state is set at 10 minutes (an interval that is the same as the sampling interval c illustrated in Fig. 6). On the other hand, in the transmitter-receiver 14, the sensor side control unit 22 reads out a program for measuring the blood sugar level and the sampling interval information from the sensor side storage unit 24, thereby allowing blood sugar level detection to be performed by the sensor 12.

After the initial setting, detection of the blood sugar level of the user by the sensor 12 is started on the basis of control by the sensor side control unit 22 (step S12).

At this point, the blood sugar monitoring system 10 determines, in the transmitter-receiver 14, whether or not there is an instruction for changing the sampling interval information from the monitoring device 16 (step S13). When there is an instruction for changing the sampling interval information, the process proceeds to step S14. When there is no instruction for changing the sampling interval information, the process proceeds to step S15 without performing step S14.

In step S14, sampling interval information is transmitted from the monitoring device 16 to the transmitter-receiver 14, and the sensor side control unit 22 changes the blood sugar level sampling interval. In this case, in the monitoring device 16, selection (setting) of pattern data based on a carbohydrate value has already been performed.

Then, the sensor 12 detects a blood sugar level, and data of the detected blood sugar level is stored in the sensor side storage unit 24 (step S15). In the blood sugar level detection, the sensor side control unit 22 performs counting based on the sampling interval information by the internal timer. Then, when the counting reaches a predetermined count, the sensor side control unit 22 supplies electric power to the light emitting element of the sensor 12, and direct measurement light to the user to optically measure the blood sugar level of the user.

Then, the blood sugar monitoring system 10 transmits the blood sugar level data stored in the sensor side storage unit 24 to the monitoring device 16 through the sensor side transmitter-receiver module 26 and the monitoring side transmitter-receiver module 34 (step S16). The blood sugar level data is stored in the blood sugar level data storage area 64 of the monitoring side storage unit 32.

Then, whether or not finishing the blood sugar level detection is determined (step S17). When continuing the blood sugar level detection, the process returns to step S13, and the processes from S13 to S16 are again performed.

On the other hand, when finishing the blood sugar level detection, the power sources are turned off in step S18 to stop the actuation of, the sensor 12, the transmitter-receiver 14, and the monitoring device 16 (step S18).

As described above, the blood sugar monitoring system 10 can measure blood sugar levels on the basis of the set sampling interval. Confirmation of display of the blood sugar level by the monitoring device 16, that is, confirmation of a measurement result can be freely performed by an operation by a doctor, a nurse, or a user during measurement of the blood sugar level and when finishing the measurement regardless of the detection of the blood sugar level.

Next, the flow of processing for setting a blood sugar level sampling interval when taking a meal will be described. As illustrated in Fig. 8, the monitoring device 16 is in a stand-by mode before starting the setting (step S20). In the stand-by mode, the function to receive blood sugar level data from the transmitter-receiver 14 and store the received blood sugar level data and the time measurement function of the timer 40 inside the monitoring device 16 are driven with low power, and data of blood sugar levels detected by the sensor 12 are continuously stored.

When a user takes a meal, the monitoring device 16 is operated by the user, and a sampling interval setting mode is started by the monitoring side control unit 30 which is operated by the control program 44 (step S21) . In this case, a guidance screen for setting the sampling interval is displayed on the display panel 36.

Then, the monitoring side control unit 30 displays a screen for inputting clock time for allowing mealtime information to be input (set) on the display panel 36 (step S22). When mealtime information is input from a user, the mealtime information is stored in the mealtime storage area 52 through the mealtime setting unit 46.

Then, the monitoring side control unit 30 displays a screen for inputting carbohydrate values for allowing carbohydrate values (information based on meal contents) to be input (set) on the display panel 36 (step S23). In the input of carbohydrate values, a plurality of carbohydrate values are individually input for respective meal contents, and the plurality of carbohydrate values for the respective meal contents are stored in the meal data storage area 54 through the meal data setting unit 48. At this point, the carbohydrate values are stored in association with the mealtime information. As already described above, a total value of carbohydrate values in one meal may be input by a user.

Then, the monitoring side control unit 30 (the sampling interval setting unit 50) reads out and adds the plurality of carbohydrate values associated with the mealtime information, and sets pattern data of the blood sugar level sampling interval on the basis of the total value of the carbohydrate values (step S24). At this point, the sampling interval data table 62 stored in the database storage area 58 is read out, and one piece of the pattern data (the first to third patterns) registered in the sampling interval data table 62 is selected.

Then, the monitoring side control unit 30 reads out mealtime information stored in the mealtime storage area 52, matches a zero base of the selected piece of pattern data to the read-out mealtime, and stores (sets) the information in the sampling interval storage area 56 as the sampling interval information (step S25).

Then, the monitoring side control unit 30 determines whether or not transmitting the sampling interval information to the transmitter-receiver 14 on the basis of the clock time measurement of the timer 40 (step S26). In this case, the monitoring side control unit 30 compares information of a performance period (clock time) contained in the set sampling interval information (pattern data) with a current clock time, and determines whether or not a currently-performed sampling interval is switched to a different sampling interval. When the currently-performed sampling interval is switched to a different sampling interval, the process proceeds to step S27. When the currently-performed sampling interval is not switched, the determination is continued.

In step S27, new sampling interval information is transmitted from the monitoring device 16 to the transmitter-receiver 14.

When the transmitter-receiver 14 receives sampling interval information from the monitoring device 16, the sensor side control unit 22 changes the blood sugar level sampling interval on the basis of the received sampling interval information (step S28). Then, the sensor side control unit 22 performs blood sugar level detection by the sensor 12 on the basis of the changed sampling interval.

It is needless to say that the contents of data transmitted and received between the monitoring device 16 and the transmitter-receiver 14 can take various forms not only transmitting sampling interval information every time when the sampling intervals are switched as described above.

For example, the blood sugar monitoring system 10 may directly transmit pattern data set by the monitoring device 16 on the basis of a carbohydrate value (information obtained by combining a plurality of kinds of sampling intervals) to the transmitter-receiver 14, and store the pattern data in the sensor side storage unit 24. The sensor side control unit 22 can detect a blood sugar level using the stored pattern data. Further, since the transmitter-receiver 14 is provided with a timer (not illustrated) inside thereof, the timer measuring clock time, it is possible to perform switching between different sampling intervals according to clock time measurement of the timer, and thereby perform blood sugar level sampling according to pattern data. In this manner, merely by transmitting pattern data once from the monitoring device 16, it is possible to detect blood sugar levels at the set sampling interval. Therefore, transmission and reception of data is simplified.

As described above, the blood sugar monitoring system 10 according to the present embodiment can easily change the setting of sampling interval using a carbohydrate value by the monitoring device 16 which displays a result of blood sugar level measurement to thereby change the interval of blood sugar level sampling performed by the sensor 12.

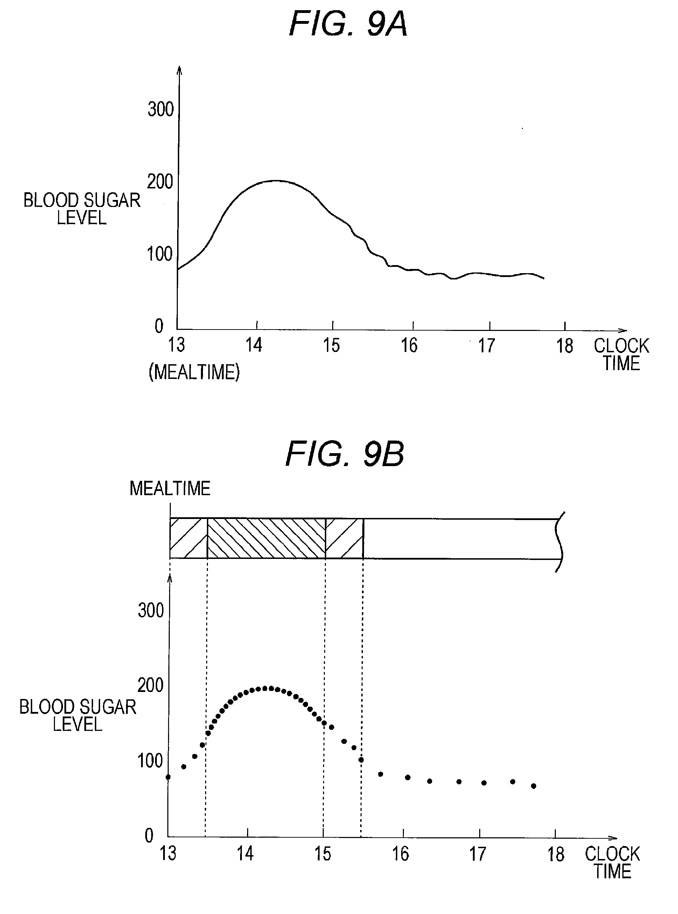

Next, as a concrete example of change of the blood sugar level sampling interval, a case where a user selects curry and rice at lunch time and finishes eating at 13: 00 will be described. Fig. 9A is a graph illustrating an example of a fluctuation curve of the blood sugar level according to the concrete example. Fig. 9B is a sample graph of blood sugar level data obtained from the fluctuation curve of Fig. 9A when changing a blood sugar level sampling interval using the blood sugar monitoring system 10. Note that the blood sugar level sampling interval illustrated in Fig. 9B is schematically illustrated, and is different from an actual sampling interval.

When a user takes the meal as described above, predetermined processing is performed according to the flow of setting the sampling interval described above (refer to Fig. 8). Specifically, 13:00 is set (stored) as mealtime information in the mealtime storage area 52, and 14 (refer to Fig. 2) is set as carbohydrate value data in one meal in the meal data storage area 54 by an operation by a user. When the two pieces of information (the mealtime information and the information based on meal contents) are input in association with each other, the monitoring side control unit 30 sets a blood sugar level sampling interval (pattern data). In this case, since the carbohydrate value of curry and rice is 14, the first pattern is selected with reference to the sampling interval data table 62 illustrated in Fig. 6. Then, the zero base of the first pattern is matched to 13:00 (the mealtime information) read out from the mealtime storage area 52, and information of the blood sugar level sampling interval based on the clock time is stored (set) in the sampling interval storage area 56.

Sampling interval information corresponding to the first pattern is transmitted to the transmitter-receiver 14 from the monitoring device 16, and the blood sugar level is detected on the basis of the sampling interval information. That is, the detection is performed at the sampling interval b of five minutes from 13:00 to 13:30, at the sampling interval a of one minute from 13:30 to 15:00, again at the sampling interval b of five minutes from 15: 00 to 15:30, and at the sampling interval c of ten minutes after 15:30.

When the carbohydrate value is a high value such as 14, it is assumed that carbohydrates are converted to blood sugar without taking long time. Therefore, a fluctuation curve in which the blood sugar level rapidly increases and shows substantially its highest peak value at approximately one hour after the meal (around 14:00) is predicted. The blood sugar monitoring system 10 can accurately grasp a fluctuation state (fluctuation curve) near the peak value of the blood sugar value by making the sampling interval short regarding blood sugar levels during the period in which the fluctuation rate is large to thereby increase the number of times of sampling.

As described above, with the blood sugar monitoring system 10 according to the present embodiment, the sampling interval setting unit 50 sets the sampling interval of the sensor 12 using a carbohydrate value, thereby making it possible to increase the number of times of blood sugar level sampling by making the sampling interval short in a period during which the blood sugar level largely fluctuates after a meal and reduce the number of times of blood sugar level sampling by making the sampling interval long in a period during which fluctuation of the blood sugar level is small. Accordingly, it is possible to accurately grasp the blood sugar level in a period during which the fluctuation rate is large. In addition, in a period during which the fluctuation rate of the blood sugar level is small, it is possible to reduce the number of times of detection to reduce the processing load of the detection result, thereby reducing power consumption. In particular, in an optical measurement method in which power is supplied on the basis of a sampling interval, power consumption in the sensor 12 can be significantly reduced. That is, a fluctuation state of the blood sugar level can be efficiently monitored, and excellent blood sugar management can thereby be performed.

In this case, by timely changing the sampling interval of the sensor 12 on the basis of mealtime information, the blood sugar level which increases after a meal and has a large fluctuation rate around a predetermined time near its peak value can be detected at a short sampling interval. As a result, it is possible to improve the monitoring accuracy during a period that is important for blood sugar management.

Further, by displaying the carbohydrate value database 60 by the display panel 36, a user can easily uses carbohydrate values based on meal contents displayed thereon. In this case, when the meal data setting unit 48 sets a carbohydrate value in response to selection of a meal content displayed by the display panel 36, a user can use a carbohydrate value only by selecting a meal content. Therefore, it is possible to omit an operation of converting meal contents to carbohydrate values by a user him/herself, and thereby more easily set the blood sugar level sampling interval.

Further, by selecting one piece from a plurality of pieces of pattern data on the basis of a total value of carbohydrate values, it is possible to easily change the sampling interval according to the selected piece of pattern data based on the carbohydrate value. In addition, it is possible to detect the blood sugar level by setting the shortest sampling interval in one piece of pattern data so as to match a predetermined time before and after its peak value in which the fluctuation rate of the blood sugar level is large.

It is needless to say that the present invention is not limited to the above embodiment, and can take various configurations without departing from the scope of the invention.

## Claims

1. An analyte monitoring system (10) comprising:
a detection means (12) embedded inside the body of a user, the detection means (12) detecting the concentration of an analyte on the basis of a sampling interval;
a meal data setting means (48) setting information based on meal contents; and
a sampling interval setting means (50) setting the sampling interval of the detection means (12) using the information based on meal contents set by the meal data setting means (48), wherein
the analyte monitoring system (10) continuously measures an analyte by the detection means (12).

2. The analyte monitoring system (10) according to claim 1, further comprising a mealtime setting means (46) setting mealtime information, wherein
the sampling interval setting means (50) changes the sampling interval of the detection means (12) on the basis of mealtime information set by the mealtime setting means (46).

3. The analyte monitoring system (10) according to claim 1, wherein the information based on meal contents is a carbohydrate value indicating the amount of carbohydrates in each of the meal contents.

4. The analyte monitoring system (10) according to claim 3, further comprising:
a database storage means (58) storing the carbohydrate value for each of the meal contents as a database (60); and
a display means (36) displaying a meal content with which the carbohydrate value is associated.

5. The analyte monitoring system (10) according to claim 4, wherein, in response to selection of a meal content displayed by the display means (36), the meal data setting means (48) sets the carbohydrate value of the selected meal content.

6. The analyte monitoring system (10) according to claim 3, further comprising a sampling pattern storage means (58) capable of storing a plurality of pieces of pattern data combining a plurality of kinds of sampling intervals, wherein
the sampling interval setting means (50) calculates a total value of the carbohydrate values in one meal, and selects one of the plurality of pieces of pattern data on the basis of the total value.

7. The analyte monitoring system (10) according to claim 6, wherein the analyte is a blood sugar level.

8. The analyte monitoring system (10) according to claim 7, wherein in each of the plurality of pieces of pattern data, a period during which each of the plurality of kinds of sampling intervals is performed is set depending on the vicinity of a peak value in which the blood sugar level is predicted to be highest after a meal on the basis of the carbohydrate value.
